# EUROPÄISCHE PATENTSCHRIFT

(11) **EP 0 734 232 B1**
(45) Veröffentlichungstag und Bekanntmachung des Hinweises auf die Patenterteilung: **09.09.1998**
(21) Anmeldenummer: 94919645.5
(22) Anmeldetag: 20.06.1994
(51) Int. Cl.: A61B 17/60

(54) **UNILATERALER FIXATEUR**
UNILATERAL FIXATION DEVICE
FIXATEUR UNILATERAL

(30) Priorität: 17.12.1993 DE 9319433 U
(43) Veröffentlichungstag der Anmeldung: 02.10.1996
(73) Patentinhaber: Reckert, Lothar, 29587 Natendorf (DE)
(72) Erfinder: Reckert, Lothar, 29587 Natendorf (DE)
(74) Vertreter: Fleck, Thomas, Dr. Dipl.-Chem.
(86) Internationale Anmeldenummer: EP9402010
(87) Internationale Veröffentlichungsnummer: WO9516401

(56) Entgegenhaltungen:
- EP-A- 0 315 215
- EP-A- 0 424 292
- WO-A-91/01201
- DE-C- 3 807 346
- DE-U- 9 319 433

## Beschreibung

Die Erfindung betrifft einen unilateralen Fixateur zur äußeren Repositionierung und Stabilisierung gebrochener Knochen, insbesondere großer und kleiner Röhrenknochen, mit einem in der Länge veränderbaren Mittelteil, dessen Enden jeweils über ein Kugelgelenk mit einer Klemmbacke verbunden sind, die jeweils mehrere quer zur Längsachse verlaufende Bohrungen zur entsprechenden Aufnahme und Fixierung von Knochennägeln aufweist.

Ein solcher Fixateur ist aus der EP-B-0 011 258 bekannt. Der bekannte Fixateur zeichnet sich durch eine relativ kompliziert aufgebaute Druck- und Zugeinrichtung aus, die an den relativ zueinander verschiebbaren Teilen des Mittelteiles angreift sowie eine Spindel umfaßt, mittels der die Länge des Fixateurs verändert werden kann. Diese bekannte Konstruktion ist aus Aluminium und Edelstahlteilen zusammengesetzt. Der Mittelkörper besteht aus Aluminium, der gegenüber hochfestem Stahl oder anderen Legierungen eine geringe Festigkeit aufweist und aus diesem Grunde sehr stark dimensioniert werden muß. Die Schraube zum Festsetzen des Mittelteiles in axialer Richtung ist aus Edelstahl, ebenfalls die dazugehörige Mutter, die in den Aluminium-Körper des Mittelteiles eingesetzt ist. Das Kugelgelenk ist aus Edelstahlkomponenten hergestellt und mit den Aluminium-Teilen verbunden. Die Klemmbacken für die Aufnahme der Knochennägel sind ebenfalls aus Aluminium, während die Schrauben für die Festsetzung der Knochennägel aus Edelstahl bestehen. Das Kugelgelenk wird durch einen Exzenterbolzen aus Edelstahl festgesetzt, der in Aluminium gelagert ist. Bei längerer Benutzung des bekannten Fixateurs kommt es an den Verbindungsstellen (Stahl-Aluminium) zu Materialermüdungen und Materialbrücken. Die Festsetzung der Kugelgelenke wird bei längerem Gebrauch nicht mehr ausreichend sichergestellt da es im hochbelasteten Bereich (Exzenterbolzen-Lagerung) zu plastischen Verformungen des Materials kommt. Die Knochennägel, die aus hochfestem Implantatstahl gefertigt sind, finden in den weicheren Aluminium-Klemmbacken keinen ausreichenden Halt. Hinzu kommt, daß die Aluminium-Klemmbacken jeweils mehrteilig ausgebildet sind, wodurch ebenfalls Schwachstellen begründet werden. Besonders bei dynamischer Belastung kommt es zu einer Lockerung und Verschiebung der Montage, die sich auf die feste Halterung des Knochens und somit auf die Heilung nachhaltig auswirkt. Das Gewicht der vorbekannten Vorrichtung ist relativ groß und führt zu Muskelirritationen. Insgesamt stellt sich die bekannte Konstruktion als relativ aufwendig und dementsprechend schwierig in der Handhabung dar. Darüber hinaus ist keine dauerhafte Festigkeit gewährleistet.

Ferner ist aus der EP-A-424 292 ein Fixateur bekannt, bei dem zwei Teile starr verbunden sind.

Der vorliegenden Erfindung liegt die Aufgabe zugrunde, einen unilateralen Fixateur der eingangs genannten Art so zu verbessern, daß auch bei längerer Benutzung eine absolut sichere Festlegung der einzelnen Teile gewährleistet ist. Des weiteren soll sich der erfindungsgemäße Fixateur durch eine einfache Konstruktion auszeichnen, die eine minimale Anzahl von Bauteilen sowie entsprechend geringes Gewicht besitzt.

Diese Aufgabe wird erfindungsgemäß durch die kennzeichnenden Merkmale des Anspruches 1 gelöst. Im Vergleich zum Stand der Technik zeichnet sich die erfindungsgemäße Konstruktion durch eine erheblich kleinere Dimensionierung der einzelnen Komponenten aus. Dementsprechend kann der Platzbedarf und das Gewicht des Fixateurs reduziert werden, und zwar um etwa 50% im Vergleich zum Fixateur gemäß der EP-B-0 011 258.

Daneben bestehen sämtliche Bauteile, auch das bzw. die Kugelgelenke erfindungsgemäss aus einer hochfesten Titanlegierung. Die anhand des oben genannten Standes der Technik erläuterten Materialprobleme treten dementsprechend beim erfindungsgemäßen Fixateur nicht mehr auf. Es ist auch bei längerer Benutzung eine absolut sichere Festlegung des Fixateurs gewährleistet. Eine besonders vorteilhafte Titanlegierung ist in Anspruch 2 angegeben.

Anspruch 5 beschreibt eine bevorzugte Ausführungsform einer Kugelgelenk-Verbindung zwischen Mittelteil und Klemmbacken. Hinsichtlich einer sicheren Festlegung bzw. Fixierung des Kugelgelenks wird insbesondere auf die Maßnahmen nach Anspruch 6 verwiesen, wonach der Radius der sphärisch gewölbten Kugelkopf-Klemm- und -Stützflächen im Bereich des Scheitels derselben kleiner ist als im übrigen Bereich, oder in Richtung zum Scheitel hin kontinuierlich abnimmt. Durch diese Maßnahme wird eine punktförmige Festlegung des Kugelkopfes vermieden; statt dessen wird stets ein ringförmiger Kontakt zwischen Kugelkopf und zugeordneter Klemmfläche sichergestellt, der bei Ausbildung der Kugelgelenk-Teile aus einer hochfesten Titanlegierung sogar in einer Art Kaltverschweißung zwischen Kugelkopf und zugeordneter Klemm- bzw. Stützfläche führt, die sich bei Bedarf auch wieder lösen läßt. Dementsprechend zeichnet sich der bekannte Fixateur als äußerst funktionssicher, vor allem dauerhaft funktionssicher aus.

Um den Reibschluß zwischen Kugelkopf und zugeordneter Klemm- bzw. Stützfläche zusätzlich zu erhöhen, können die vorgenannten Teile noch mit Diamantstaub beschichtet sein.

Ein besonders vorteilhafter Kugelgelenk-Klemm-Mechanismus ist noch in Anspruch 8 angegeben. Dieser Klemm-Mechanismus ist besonders einfach in der Konstruktion, aber dennoch höchst sicher in der Funktion. Des weiteren ist er besonders leicht zugänglich und dementsprechend handhabbar. In überraschender Weise lassen sich trotz des Klemm-Mechanismus gemäß Anspruch 8 die Klemmbacken, die den vorgenannten Klemm-Mechanismus halten, jeweils einteilig ausbilden, wobei die Querbohrungen zur Aufnahme und Fixierung der Knochennägel sich jeweils seitlich an der axialen Gewindebohrung für die Klemmschraube des in Anspruch 9 beschriebenen Mechanismus vorbeierstrecken.

Trotz dieser Konstruktion ist es nicht erforderlich, die Klemmbacken größer als beim Stand der Technik zu dimensionieren. Dies ist durchaus überraschend und nicht zuletzt bedingt durch die Ausbildung der Klemmbacke aus hochfester Titanlegierung. Für den Operateur ist darüber hinaus von Bedeutung, daß die Klemmbacken bei der erfindungsgemäßen Konstruktion jeweils einteilig ausgebildet sind, wodurch deren Handhabung erheblich erleichtert wird.

Die Ausführungsform nach Anspruch 12 hat zur Folge, daß eine gesonderte Druck- und Zugeinrichtung für die Längenveränderung des Mittelteils vermieden werden kann. Insbesondere ist kein gesonderter Distraktor erforderlich, der an den beiden Rundstäben zur Veränderung der Länge des Mittelteils angreift. Die Längenveränderung und Fixierung der Mittelteil-Länge erfolgt durch die in den Ansprüchen 13 und 14 beanspruchten Muttern, die eine Art Doppelfunktion besitzen. Zum einen dienen sie zur Veränderung der Länge des Mittelteils; zum anderen läßt sich mittels der Muttern die eingestellte Länge des Mittelteils fixieren.

Der erfindungsgemäße Fixateur läßt sich auch als Bohrschablone verwenden, wodurch zusätzlich ein schnelleres Operieren möglich wird.

Nachstehend werden bevorzugte Ausführungsbeispiele eines erfindungsgemäßen Fixateurs anhand der beigefügten Zeichnung näher erläutert. Es zeigen:
- Fig. 1: eine schematische Vorderansicht einer ersten Ausführungsform eines erfindungsgemäßen Fixateurs mit sechs Knochennägeln, die an einem teilweise unterbrochenen Knochen angebracht sind;
- Fig. 2: einen teilweisen senkrechten Mittelschnitt des Fixateurs gemäß Fig. 1, wobei der untere Teil mit Kugelgelenk und Klemmbacke um 90° gedreht und nicht im Schnitt dargestellt ist;
- Fig. 3: einen vergrößerten Schnitt des Kugelgelenks;
- Fig. 4: eine zweite Ausführungsform eines erfindungsgemäßen Fixateurs im Längsschnitt;
- Fig. 5: den Fixateur gemäß Fig. 4 im Querschnitt längs Linie A-A in Fig. 4:
- Fig. 10: eine weitere Alternative eines Fixateurs gemäß Erfindung mit einem Mittelteil entsprechend dem in Fig. 1 bzw. Fig. 2; und
- Fig. 11: einen dem Fixateur gemäß den Fig. 1 und 2 zugeordneten Distraktor in Draufsicht.

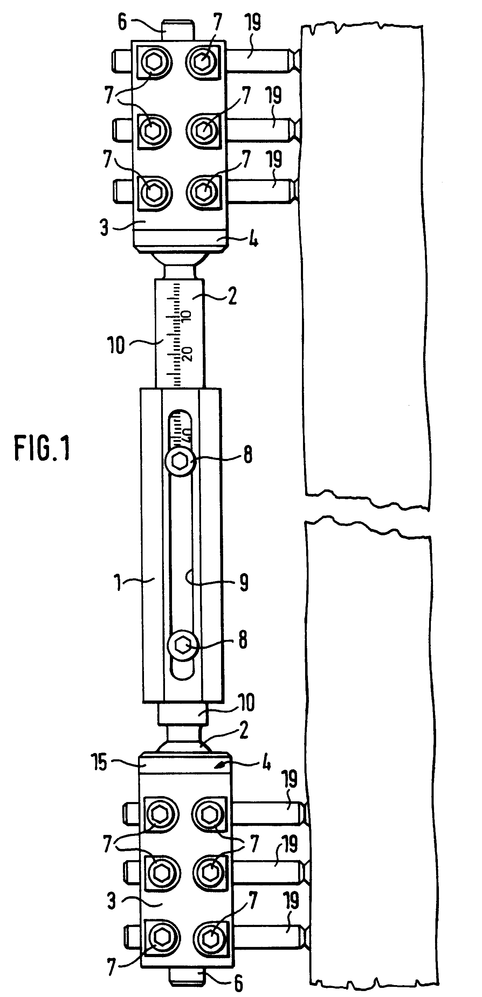

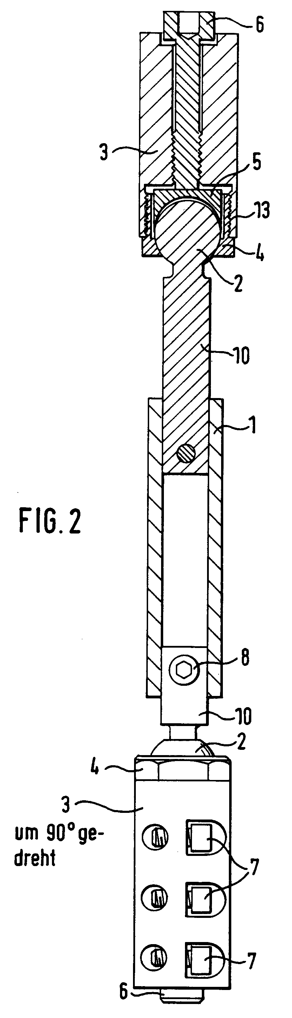

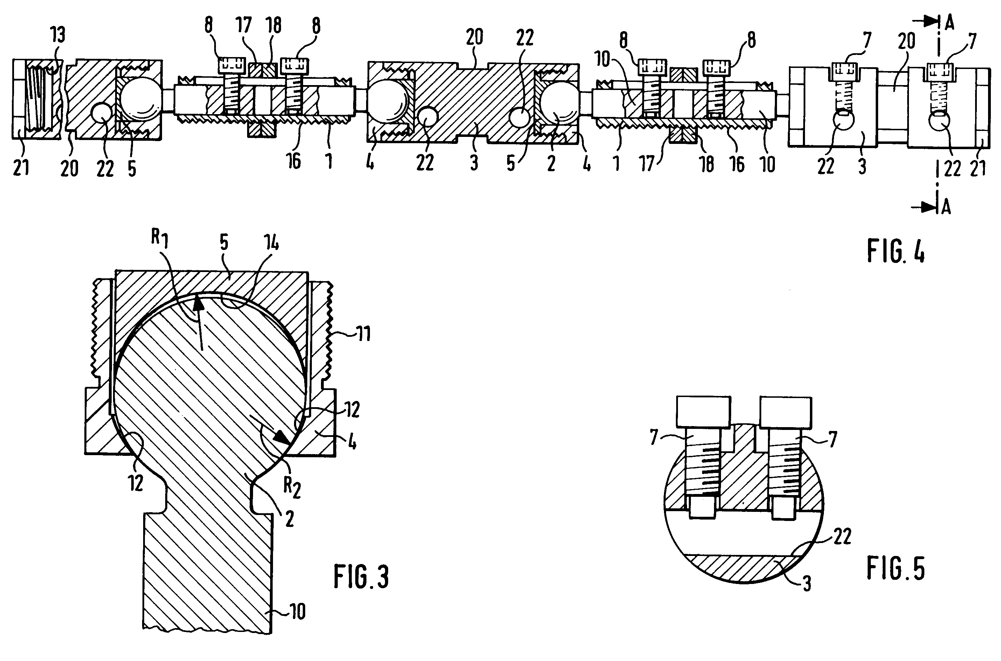

Die Fig. 1 bis 3 sind in unterschiedlichem Maßstab gehalten. Gleiche Bezugsziffern entsprechen gleichen oder gleichwertigen Teilen. Die in den Fig. 1 bis 3 dargestellte Ausführungsform eines Fixateurs weist einen Mittelkörper auf, der aus einer länglichen Hülse 1 mit Langloch besteht, in den teleskopartig an beiden Seiten ein Rundstab mit Kugelkopf 2 eingefügt ist, der am Ende seines zylindrischen Teils mit einem Gewinde quer zur Stabachse versehen ist. Das erwähnte Langloch in der Hülse 1 ist mit der Bezugsziffer 9 gekennzeichnet. Durch dieses Langloch hindurch lassen sich mit zwei Schrauben 8, deren Kopf jeweils mit einem Innensechskant versehen ist, die beiden Rundstäbe mit der Hülse 1 verbinden. Die beiden Rundstäbe sind jeweils mit der Bezugsziffer 10 gekennzeichnet. Der Kopf jeder Schraube 8 stützt sich beim Anziehen auf einer außen angefrästen Fläche der Hülse 1 ab. Sind die Schrauben 8 gelöst, lassen sich die Rundstäbe 10 samt Kugelkopf 2 in axialer Richtung verschieben, bis sie am Ende des Langloches 9 anschlagen. Gegen Rotation relativ zur Hülse 1 sind die Rundstäbe 10 durch die Schrauben 8 gesichert. Durch Anziehen der beiden Schrauben 8 wird die Länge des Mittelteils fixiert. Entsprechend den Fig. 2 und 3 ist der Kugelkopf 2 eines jeden Rundstabes 10 innerhalb eines mit Außengewinde 11 versehenen hülsenartigen Kugelklemmstücks 4 mit einer ringförmig nach innen vorspringenden, sphärisch gewölbten Kugel-Stützfläche 12 plaziert, wobei das Kugelklemmstück 4 mit dem Kugelkopf 2 voran in eine zugeordnete Gewindebohrung 13 einer noch näher zu beschreibenden Klemmbacke 3 einschraubbar ist, und zwar unter Zwischenschaltung einer mit einer sphärisch gewölbten Kugelkopf-Klemmfläche 14 versehenen Kugeldruckscheibe 5 zwischen Kugelkopf 2 und Klemmbacke 3, so wie dies im Teil-Längsschnitt der Fig. 2 dargestellt ist. Das Kugelklemmstück 4 ist mit einem Außensechskant 15 versehen, so daß es mittels eines passenden Maulschlüssels fest in die Klemmbacke 3 einschraubbar ist. Zur besseren Fixierung bzw. Feststellung des Kugelgelenks ist der Radius R₁ der sphärisch gewölbten Kugelkopf-Klemmfläche 14 der Kugeldruckscheibe 5 und/oder der Radius R₂ der sphärisch gewölbten Kugel-Stützfläche 12 innerhalb des hülsenartigen Kugelklemmstücks 4 im Bereich des Scheitels, d. h. im Bereich der Mittenachse von Kugeldruckscheibe 5 bzw. Kugelklemmstück 4 kleiner als im übrigen Bereich. Vorzugsweise nehmen die vorgenannten Radien in Richtung zum erwähnten Scheitel hin ab. Damit wird eine ringförmige Kontaktfläche zwischen Kugelkopf 2 und Kugelkopf-Klemmfläche 14 bzw. Kugelkopf-Stützfläche 12 hergestellt. Wie bereits eingangs erwähnt, können Kugelkopf 2 und/oder die Kugelkopf-Klemmflächen bzw. Kugelkopf-Stützflächen jeweils mit Diamantstaub beschichtet sein, um einen erhöhten Reibschluß zwischen den genannten Flächen zu erhalten.

Die bereits erwähnten Klemmbacken 3, die bei dem dargestellten Ausführungsbeispiel jeweils drei quer zur Längsachse verlaufende Bohrungen zur Aufnahme und Fixierung von Knochennägeln 19 aufweisen, sind jeweils als einstückiges rundstabartiges Bauteil ausgeführt, an dessen einer Stirnseite die Gewindebohrung 13 zur Aufnahme der Kugeldruckscheibe 5, des Kugelgelenkkopfes 2 und des hülsenartigen Kugelklemmstücks 4 ausgebildet ist und durch das sich eine auf die Kugeldruckscheibe 5 einwirkende Klemmschraube 6 axial hindurcherstreckt. Die erwähnten Querbohrungen zur Aufnahme und Fixierung der Knochennägel 19 erstrecken sich jeweils seitlich an der axialen Gewindebohrung für die Klemmschraube 6 vorbei. Durch Anziehen der Klemmschraube 6 wird die Kugeldruckscheibe 5 gegen den Kugelkopf 2 gedrückt, der sich auf der Gegenseite an der ringförmigen Kugel-Stützfläche 12 des hülsenförmigen Kugelklemmstücks 4 abstützt. Auf diese Weise wird das beschriebene Kugelgelenk festgesetzt. Beim Anziehen der Schraube 6 sollte zum Gegenhalten der erwähnte Maulschlüssel am Außensechskant 15 des Kugelklemmstücks 4 angesetzt werden, um keine Momente auf die Montage zu übertragen.

Das in Fig. 2 unten, nicht im Schnitt dargestellte und um 90° gedrehte Bauteil 3 zeigt, wie die Knochennägel 19 aufgenommen und fixiert werden. In jeder Klemmbacke 3 befinden sich drei Querbohrungen, in denen die Knochennägel 19 aufgenommen werden. Senkrecht zu jeder Querbohrung sind zwei Gewinde geschnitten, die bis in die Querbohrungen reichen (siehe Fig. 1), und durch die jeweils zwei Schrauben 7 zur Fixierung der Knochennägel 19 einschraubbar sind.

Vorzugsweise bestehen sämtliche Bauteile des beschriebenen Fixateurs aus einer hochfesten Titanlegierung. Insbesondere ist die verwendete Titanlegierung durch folgende Bestandteile gekennzeichnet:
Aluminium 5,5 - 7,0%; Molybdän 2,8 - 3,8%; Zirkon 1,5 - 2,0%; Eisen 0,25%; Kohlenstoff 1,0%; Silicium 0,15 - 0,25%; Rest Titan.

Die beschriebenen Fixateure können eine Länge von 20,5 cm oder alternativ 13,5 cm besitzen und um 5 bzw. 4 cm verlängerbar sein. Letztlich hängt dies von der Länge bzw. Größe des zu stabilisierenden Knochens ab. Des weiteren sind die Abmessungen der Kugelgelenke so gewählt, daß die Klemmbacken 3 zur Längsachse bis zu 20° beweglich sind. Die erwähnten Querbohrungen in den Klemmbacken 3 zur Aufnahme und Fixierung der Knochennägel 19 weisen vorzugsweise einen Durchmesser von 6,0 bzw. 4,0 mm auf entsprechend der unterschiedlichen Größe bzw. Länge des Fixateurs.

In den Fig. 4 und 5 ist ein abgewandeltes Ausführungsbeispiel eines erfindungsgemäß ausgebildeten Fixateurs dargestellt, wobei gleiche oder gleichwertige Teile, die anhand der Fig. 1 bis 3 bereits beschrieben worden sind, bei dem Ausführungsbeispiel nach den Fig. 4 und 5 dieselben Bezugsziffern besitzen.

Die Ausführungsform nach den Fig. 4 und 5 zeichnet sich zum einen dadurch aus, daß sie im Vergleich zu der Ausführungsform nach den Fig. 1 bis 3 zwei Mittelteile in Form von jeweils zwei Rundstäbe aufnehmenden Hülsen 1 umfaßt. Dementsprechend weist diese Ausführungsform auch drei Klemmbacken 3 auf, die mit den vorgenannten Mittelteilen jeweils gelenkig (Kugelgelenke) verbunden sind. Des weiteren ist gegenüber der erstgenannten Ausführungsform unterschiedlich, daß die die Rundstäbe 10 aufnehmenden Hülsen 1 jeweils ein Außengewinde 16 aufweisen, auf das zwei Muttern, insbesondere Sechskantmuttern 17, 18 aufgeschraubt sind. Die beiden Sechskantmuttern 17, 18 befinden sich jeweils zwischen den beiden in die Rundstäbe 10 eingeschraubten Mitnehmer- und Fixierschrauben 8. Dementsprechend können nach Montage des Fixateurs die Sechskantmuttern 17, 18 in Anlage an die Schrauben 8 geschraubt werden, um diese dann bei Bedarf unter entsprechender Mitnahme der zugeordneten Rundstäbe 10 zunehmend auf Distanz voneinander zu schrauben. Dementsprechend haben die Sechskantmuttern 17, 18 eine Art Doppelfunktion. Zum einen dienen sie als sogenannter Distraktor; zum anderen lassen sich damit die Positionen der Schrauben 8 und damit der zugeordneten Rundstäbe 10 arretieren. Grundsätzlich ist es auch ausreichend, nur eine Sechskantmutter 17 oder 18 zwischen den beiden Schrauben 8 auf der Hülse 1 zu plazieren. Diese eine Sechskantmutter dient dann als Distraktor in Zuordnung zu nur einer der beiden Schrauben 8, während die andere Schraube 8 in der anhand der Fig. 1 und 2 beschriebenen Weise eingesetzt wird.

Die Klemmbacken 3 weisen bei der Ausführungsform nach den Fig. 4 und 5 zusätzlich noch Ansatzflächen, insbesondere einen Außensechskant 20 für einen Maulschlüssel auf, um das Kugelgelenk zwischen Klemmbacke 3 und dem jeweils zugeordneten und ebenfalls mit einem Außensechskant 15 versehenen Kugelklemmstück 4 durch gegenseitiges Verdrehen dieser beiden Teile fixieren zu können. Die Klemmbacken 3 weisen nämlich nach dem Ausführungsbeispiel nach den Fig. 4 und 5 keine auf die Kugeldruckscheibe 5 einwirkende Klemmschraube 6 auf. Dementsprechend sind auch die äußeren Gewindebohrungen 13 und die beiden äußeren Klemmbacken 3 durch Schraubdeckel 21 verschlossen, die ebenfalls einen Außensechskant entsprechend dein Außensechskant 15 am Kugelklemmstück 4 aufweisen.

In Fig. 5 ist die Funktion der Knochennagel-Klemmschrauben 7 gut erkennbar. Die Querbohrungen für die Knochennägel 19 in den Klemmbacken 3 sind in den Fig. 4 und 5 mit der Bezugsziffer 22 gekennzeichnet. Entsprechende Querbohrungen sind in den Klemmbacken 3 der Ausführungsform nach den Fig. 1 bis 3 vorgesehen.

Die Kugelgelenke der Ausführungsform gemäß den Fig. 4 und 5 sind in gleicher Weise wie anhand des Ausführungsbeispieles nach den Fig. 1 bis 3, insbesondere wie anhand der Fig. 3 beschrieben, ausgeführt. Insofern wird auf die entsprechende Beschreibung des Ausführungsbeispieles nach den Fig. 1 bis 3 verwiesen. Dies gilt insbesondere für das anhand der Fig. 3 dargestellte Radien-Verhältnis von Kugelkopf-Klemmfläche 14 und Kugelkopf-Ringstützfläche 12.

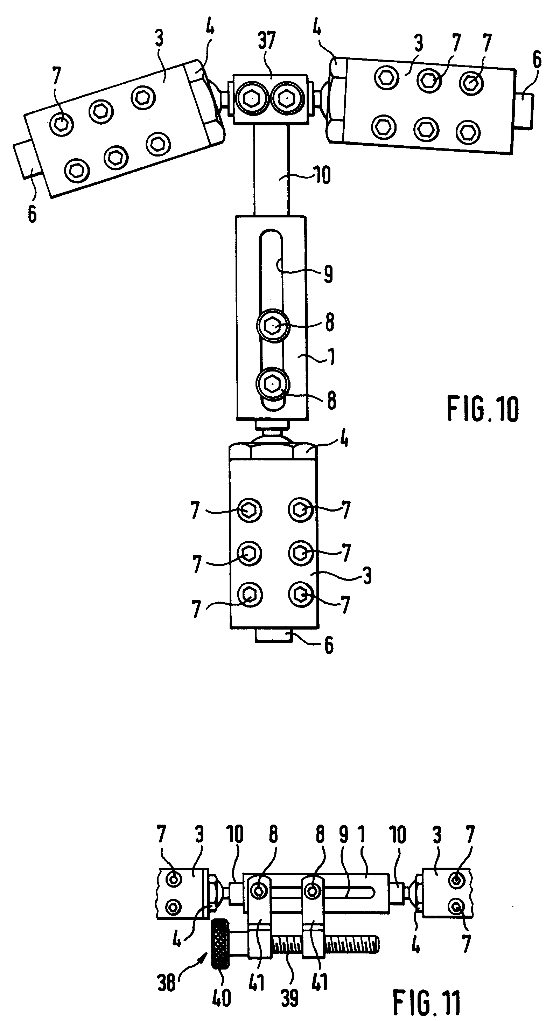

Das Ausführungsbeispiel gemäß Fig. 10 entspricht hinsichtlich der Grundkonstruktion demjenigen gemäß den Fig. 1 bis 3.

Gegenüber diesem Ausführungsbeispiel besteht im wesentlichen nur der Unterschied, daß am freien äußeren Ende des einen Rundstabes 10 statt eines Kugelkopfes ein Quersteg 37 ausgebildet ist, dessen freie Enden jeweils einen Kugelkopf 2 der beschriebenen Art tragen, so daß daran zwei Klemmbacken 3 entsprechend Fig. 10 anschließbar sind. Der Fixateur gemäß Fig. 10 umfaßt also insgesamt drei Klemmbacken 3, die zusammen mit den längenveränderbaren Mittelteil 1 eine T-förmige Gesamtkonstruktion definieren. In gleicher Weise ist auch eine L-förmige, Y-förmige, Doppel-T-förmige oder X-förmige Gesamtkonstruktion denkbar.

Anhand der Fig. 11 sei noch auf eine besondere Konstruktion eines Distraktors 38 in Zuordnung zu dem Ausführungsbeispiel nach den Fig. 1 bis 3 und 10 hingewiesen. Dieser Distraktor 38 besteht aus einer Spannschraube 39 mit Rändelkopf 40 sowie zwei Querlaschen 41, an deren einem Ende jeweils eine Bohrung für den Durchtritt der Spannschraube 39 ausgebildet ist, während an den anderen Enden sich senkrecht dazu erstreckende Bohrungen zur Aufnahme der Schrauben 8 bzw. deren Köpfe befinden. Die dem Rändelkopf 40 der Spannschraube 39 nähergelegene Querlasche 41 besitzt lediglich eine Durchgangsöffnung für die Spannschraube 39, in der die Spannschraube 39 frei drehbar gelagert ist, ohne daß eine axiale Verschiebung der Querlasche 41 stattfindet. Die Querlasche 41 ist also nahe des Rändelkopfes 40 der Spannschraube 39 unverschiebbar gehalten. Die andere Querlasche, d. h. die dem distalen Ende der Spannschraube 39 nähergelegene Querlasche 41 weist ein Innengewinde in Zuordnung zu dem Gewinde der Spannschraube 39 auf, so daß bei Verdrehen der Spannschraube 39 diese Querlasche relativ zu der dem Rändelkopf 40 nähergelegenen Querlasche axial bewegbar ist. Dementsprechend kann nach Ansetzen der Querlaschen 41 an den über die Hülse 1 vorstehenden Köpfen der den Rundstäben 10 zugeordneten Schrauben 8 durch Verdrehen der Spannschraube 39 der Abstand der Schrauben 8 und damit der jeweils zugeordneten Rundstäbe 10 voneinander verändert werden. Die die Köpfe der Schrauben 8 aufnehmenden Bohrungen in den Querlaschen 41 erstrecken sich bei dem dargestellten Ausführungsbeispiel jeweils senkrecht zu den Flachseiten der Querlaschen 41. Selbstverständlich muß zur Funktion des Distraktors 38 zumindest eine der beiden Schrauben 8 gelöst werden.

Es sei an dieser Stelle auch noch bemerkt, daß die Rundstäbe 10 unterschiedliche Länge aufweisen können. Dementsprechend ist durch Austauschen eines kürzen Rundstabes gegen einen etwas längeren Rundstab eine Erweiterung der Gesamtlänge des Fixateurs möglich. In umgekehrter Weise läßt sich die maximale Gesamtlänge des Fixateurs verkürzen.

Wie oben ausgeführt, dienen die Klemmbacken 3 zur Aufnahme von sogenannten Knochennägeln. Statt der Knochennägel können in den Klemmbacken 3 auch sogenannte Schanz-Schrauben bzw. Kirschnerdrähte fixiert werden.

Die beschriebenen Fixateure dienen zur schnellen Versorgung traumatisierter Patienten und aller Instabilitäten an Knochen, insbesondere langen Röhrenknochen. Sie zeichnen sich durch einfache Handhabung, hohe Stabilität und geringes Eigengewicht aus. Sie bestehen aus nur wenigen Einzelteilen und sind dadurch leicht zerlegbar. Alle beweglichen Teile des Fixateurs können mit nur wenigen Innensechskant-Schrauben arretiert werden. Die folgende Operationstechnik kommt z. B. zur Anwendung:

Stichincision der Haut in üblicher Weise und Eröffnen der Weichteile bis zum Periost mit einem stumpfen Instrument. Nun wird grob reponiert und das Bohrloch für eine erste Schanz-Schraube im proximalen Hauptfragment vorbereitet.

Vor dem ersten Bohrvorgang durch den Fixateur, der als Bohrschablone dient, wird dieser gerade und auf eine mittlere Länge eingestellt, um eine eventuelle Distraktion oder Kompression zu ermöglichen.

Je nach Bohrerstärke werden eine bzw. zwei ineinandergeschobene Bohrbuchsen passenden Durchmessers in den Fixateur eingesetzt, mit einer vorhandenen Innensechskant-Schraube leicht festgesetzt und mit dem Fixateur, der vorerst als Griff für die Bohrbuchse dient, bis auf den Knochen geführt.

Der Fixateur wird parallel zum Knochen ausgerichtet und der erste Bohrvorgang durchgeführt. Der Bohrer und die innere Bohrbuchse werden entfernt und die erste Schanz-Schraube eingesetzt.

Anschließend erfolgt die Ausrichtung des Fixateurs in der Längsachse und Einbringung der zweiten Schanz-Schraube in gleicher Weise in das distale Fragment. Anschließend werden die Schanz-Schrauben mit jeweils zwei Innensechskant-Schrauben 7 fixiert und parallel zur ersten und zweiten Schanz-Schraube eingebracht.

Danach erfolgt die endgültige Fein-Reposition der Fraktur sowie Fixation der Kugelgelenke und des teleskopierbaren Mittelteiles. Das Ausführungsbeispiel nach den Fig. 1 bis 3 läßt sich, wie leicht erkannt werden kann, mit nur drei Schrauben, nämlich den Schrauben 6 und 8 arretieren. In ähnlich einfacher Weise erfolgt die Arretierung bei dem Ausführungsbeispiel gemäß den Fig. 4 und 5. Das gleiche gilt für das Ausführungsbeispiel nach Fig. 10 sowie auch für das Ausführungsbeispiel nach den Fig. 6 bis 9, wobei dort die beiden Schrauben 8 durch die Spannschraube bzw. Spindel 28 ersetzt sind. Insofern ergibt sich handhabungstechnisch eine zusätzliche Erleichterung.

Die Laschen 29 bzw. 30 der im Zusammenhang mit der Ausführungsform nach den Fig. 6 bis 9 beschriebenen Gelenkarme 24, 25 sind, wie die Fig. 6 bis 8 erkennen lassen, durch Querstege 36 miteinander verbunden unter Ausbildung eines U-Profils. Diese Ausführungsform ist herstellungstechnisch besonders einfach.

### Bezugszeichenliste

- 1: Hülse
- 2: Kugelkopf
- 3: Klemmbacke
- 4: Kugelklemmstück
- 5: Kugeldruckscheibe
- 6: Klemmschraube
- 7: Schraube
- 8: Schraube
- 9: Langloch
- 10: Rundstab
- 11: Außengewinde
- 12: ringförmige Kugelkopf-Stützfläche
- 13: Gewindebohrung
- 14: Kugelkopf-Klemmfläche
- 15: Außensechskant
- 16: Außengewinde
- 17: Sechskantmutter
- 18: Sechskantmutter
- 19: Knochennagel
- 20: Ansatzflächen (Außensechskant)
- 21: Schraubdeckel
- 22: Querbohrung
- 23: Scherengelenk
- 24: Gelenkarm
- 25: Gelenkarm
- 26: Gelenkverbindung
- 27: Gelenkverbindung
- 28: Spannschraube bzw. Spindel
- 29: Lasche
- 30: Lasche
- 31: Querbohrung
- 32: Querbohrung
- 33: Verbindungselement
- 34: Verbindungselement (Hülse)
- 35: Verbindungselement (Gewindehülse)
- 36: Quersteg
- 37: Quersteg
- 38: Distraktor
- 39: Spannschraube
- 40: Rändelkopf
- 41: Querlaschen

## Patentansprüche

1. Unilateraler Fixateur zur äußeren Repositionierung und Stabilisierung gebrochener Knochen mit einem in der Länge veränderbaren Mittelteil, dessen Enden jeweils über ein Kugelgelenk mit einer Klemmbacke (3) verbunden sind, die jeweils mehrere quer zur Längsachse verlaufende Bohrungen (22) zur entsprechenden Aufnahme und Fixierung von Knochennägeln (19) aufweisen, wobei
das aus einem Hohlprofil bestehende Mittelteil ein axial verlaufendes Langloch aufweist,
und das im Mittelteil an jedem Ende axial verschiebbar gelagerte stabartige Element durch das Langloch hindurchragende Mitnehmer aufweist, dadurch gekennzeichnet,
daß sämtliche Bestandteile, auch das Kugelgelenk, aus einer hochfesten Titanlegierung gefertigt sind.

2. Unilateraler Fixateur nach Anspruch 1, gekennzeichnet durch folgende Titanlegierung:
Aluminium 5,5 - 7,0%; Molybdän 2,8 - 3,8%;
Zirkon 1,5 - 2,0%; Eisen 0,25%; Kohlenstoff 1,0%;
Silicium 0,15 - 0,25%; Rest Titan.

3. Fixateur nach einem der Ansprüche 1 bis 2, dadurch gekennzeichnet,
daß er eine Länge von 20,5 cm oder alternativ 13,5 cm besitzt und um 5 cm bzw. 4 cm verlängerbar ist.

4. Fixateur nach einem der Ansprüche 1 bis 3, dadurch gekennzeichnet,
daß die Klemmbacken (3) aufgrund der Kugelgelenke zur Längsachse bis 20° beweglich sind.

5. Fixateur nach einem der Ansprüche 1 bis 4, dadurch gekennzeichnet,
daß jedes Kugelgelenk einen Kugelkopf (2) umfaßt, der innerhalb eines mit Außengewinde (11) versehenen hülsenartigen Kugelklemmstücks (4) mit einer ringförmig nach innen vorspringenden sphärisch gewölbten Kugelkopf-Stützfläche (12) plattiert ist, wobei das
Kugelklemmstück (4) mit dem Kugelkopf (2) voran in eine zugeordnete Gewindebohrung (13) der Klemmbacke (3) einschraubbar ist, und zwar unter Zwischenschaltung einer mit einer sphärisch gewölbten Kugelkopf-Klemmfläche (14) versehenen Kugeldruckscheibe (5) zwischen Kugelkopf (2) und Klemmbacke (3).

6. Fixateur nach Anspruch 5,
dadurch gekennzeichnet,
daß der Radius (R₁; R₂) der sphärisch gewölbten Kugelkopf-Klemmfläche (14) der Kugeldruckscheibe (5) und/oder der sphärisch gewölbten Kugelkopf-Stützfläche (12) innerhalb des hülsenartigen Kugelklemmstücks (4) im Bereich des Scheitels kleiner ist als im übrigen Bereich bzw. in Richtung zum Scheitel hin abnimmt.

7. Fixateur nach einem der Ansprüche 1 bis 6,
dadurch gekennzeichnet,
daß der Kugelkopf (2) und/oder die Kugelkopf-Klemm- bzw. -Stützflächen (14, 12) mit Diamantstaub beschichtet sind.

8. Fixateur nach einem der Ansprüche 1 bis 7,
dadurch gekennzeichnet,
daß der Kugeldruckscheibe (5) eine in die Klemmbacke (3) einschraubbare Klemmschraube (6) zugeordnet ist, mittels der auf die der Kugelkopf-Klemmfläche (14) gegenüberliegende Seite der Kugeldruckscheibe (5) einwirkbar und dementsprechend das Kugelgelenk festsetzbar ist.

9. Fixateur nach Anspruch 8,
dadurch gekennzeichnet,
daß die Klemmbacke (3) ein einstückiges, insbesondere rundstabartiges Bauteil ist, an dessen einer Stirnseite eine Gewindebohrung (13) zur Aufnahme der Kugeldruckscheibe (5), des Kugelkopfes (2) und des hülsenartigen Kugelklemmstücks (4) ausgebildet ist und durch das sich die der Kugeldruckscheibe (5) zugeordnete Klemmschraube (6) axial hindurcherstreckt, wobei die Querbohrungen (22) zur Aufnahme und Fixierung der Knochennägel (19) sich jeweils seitlich an der axialen Gewindebohrung für die Klemmschraube (6) vorbeierstrecken.

10. Fixateur nach einem der Ansprüche 1 bis 9,
dadurch gekennzeichnet,
daß jede Klemmbacke (3) drei Bohrungen (22) zur Aufnahme der Knochennägel (19) besitzt, die jeweils durch zwei Schrauben arretierbar sind.

11. Fixateur nach Anspruch 10,
dadurch gekennzeichnet,
daß die die Knochennägel (19) aufnehmenden Bohrungen (22) einen Durchmesser von etwa 6,0 mm bzw. 4,0 mm besitzen.

12. Fixateur nach einem der Ansprüche 1 bis 11,
dadurch gekennzeichnet,
daß die die Rundstäbe (10) aufnehmende Hülse (1) ein Außengewinde (16) aufweist, auf das in Zuordnung zu den mit den Rundstäben (10) verbundenen Mitnehmern, insbesondere Schrauben (8) mindestens eine, insbesondere zwei Muttern, vorzugsweise Sechskantmuttern (17, 18) aufgeschraubt sind.

13. Fixateuer nach Anspruch 12,
dadurch gekennzeichnet,
daß die Mutter(n) (17, 18) zwischen den beiden Rundstab-Mitnehmern bzw. Schrauben (8) wirksam ist bzw. sind, und zwar in Zuordnung zu jeweils einem Rundstab-Mitnehmer bzw. einer mit einem Rundstab (10) verbundenen Schraube (8).

## Claims

1. Unilateral fixation device for the external repositioning and stabilization of broken bones having a length-variable central part, whose ends are in each case connected by means of a ball joint to a gripping jaw (3), which in each case have several holes (22) extending tranversely to the longitudinal axis for the corresponding reception and fixing of fracture pins (19), in which the central part comprising a hollow profile has an axially directed slot and that in the central part the bar-like element mounted in axially displaceable manner on each end has drivers projecting through the slot, characterized in that all the components, including the ball joint, are made from a high strength titanium alloy.

2. Unilateral fixation device according to claim 1, characterized by the following titanium alloy: 5.5 to 7.0% aluminium, 2.8 to 3.8% molybdenum, 1.5 to 2.0% zirconium, 0.25% iron, 1.0% carbon, 0.15 to 0.25% silicon and the remainder titanium.

3. Fixation device according to one of the claims 1 to 2, characterized in that it has a length Of 20.5 or alternatively 13.5 cm and can be lengthened by 5 or 4 cm.

4. Fixation device according to one of the claims 1 to 3, characterized in that the gripping jaws (3) are movable as a result of the ball joints by up to 20° towards the longitudinal axis.

5. Fixation device according to one of the claims 1 to 4, characterized in that each ball joint incorporates a ball end (2), which within a sleeve-like ball clamp (4) provided with an external thread (11) is plated with an annular, inwardly projecting, spherically curved ball end support surface (12), in which the ball clamp (4) can be screwed with the ball end (2) at the front into an associated tapped hole (13) of the gripping jaw (3), accompanied by the interposing of a ball pressure disk (5) provided with a spherically curved ball end clamping surface (14) between the ball end (2) and gripping jaw (3).

6. Fixation device according to claim 5, characterized in that the radius (R₁; R₂) of the spherically curved ball end clamping surface (14) of the ball pressure disk (5) and/or the spherically curved ball end support surface (12) within the sleeve-like ball clamp (4) is smaller in the vicinity of the apex than in the remaining area or decreases towards the apex.

7. Fixation device according to one of the claims 1 to 6, characterized in that the ball end (2) and/or the ball end clamping or support surfaces (14, 12) are coated with diamond dust.

8. Fixation device according to one of the claims 1 to 7, characterized in that with the ball pressure disk (5) is associated a clamping bolt (6) which can be screwed into the gripping jaw (3) and by means of which an action can take place on the side of the ball pressure disk (5) facing the ball end clamping surface (14) and consequently the ball joint is fixed.

9. Fixation device according to claim 8, characterized in that the gripping jaw (3) is a one-piece, particularly round bar-like component, on whose one end face is formed a tapped hole (13) for receiving the ball pressure disk (5), the ball end (2) and the sleeve-like ball clamp (4) and through which extends axially the clamping bolt (6) associated with the ball pressure disk (5), the cross-holes (22) for receiving and fixing the fracture pins (19) in each case extending laterally past the axial tapped hole for the clamping bolt (6).

10. Fixation device according to one of the claims 1 to 9, characterized in that each gripping jaw (3) has three holes (22) for receiving the fracture pins (19), which are in each case arrested by two screws.

11. Fixation device according to claim 10, characterized in that the holes (22) receiving the fracture pins (19) have a diameter of approximately 6.0 mm or 4.0 mm.

12. Fixation device according to one of the claims 1 to 11, characterized in that the sleeve (1) receiving the round bars (10) has an external thread (16) on which are screwed in association with the drivers, particularly screws (8), connected to the round bars (10) at least one and in particular two nuts, preferably hexagonal nuts (17, 18).

13. Fixation device according to claim 12, characterized in that the nut or nuts (17, 18) act between the two round bar drivers or screws (8) in association with in each case a round bar driver or a screw (8) connected to a round bar (10).

## Revendications

1. Fixateur unilatéral destiné au repositionnement extérieur et à la stabilisation d'os brisés, avec une partie médiane à longueur modifiable, dont les extrémités sont chacune reliées, par une articulation sphérique, à une mâchoire de serrage (3), les mâchoires de serrage présentant chacune plusieurs perçages (22) s'étendant transversalement à l'axe longitudinal, destinés à recevoir et à fixer de manière correspondante des clous d'ostéosynthèse (19), la partie médiane constituée d'un profilé creux présentant un trou oblong s'étendant axialement,
et l'élément en forme de barre, monté d'une façon permettant un déplacement axial à chaque extrémité dans la partie médiane, présentant des organes d'entraînement passant à travers le trou oblong, caractérisé en ce que l'ensemble des composants et également l'articulation sphérique sont fabriqués à partir d'un alliage de titane à résistance élevée.

2. Fixateur unilatéral selon la revendication 1, caractérisé par l'alliage de titane ci-après :
aluminium 5,5 à 7,0 %; molybdène 2,8 à 3,8 %;
zirconium 1,5 à 2,0 %; fer 0,25 %; carbone 1,0 %;
silicium 0,15 à 0,25 %; le reste étant du titane.

3. Fixateur selon l'une des revendications 1 à 2, caractérisé en ce qu'il a une longueur de 20,5 cm ou, en variante, de 13,5 cm et est prolongeable de 5 cm, respectivement 4 cm.

4. Fixateur selon l'une des revendications 1 à 3, caractérisé en ce que les mâchoires de serrage (3) sont mobiles jusqu'à un angle de 20° par rapport à l'axe longitudinal, du fait de la présence des articulations sphériques.

5. Fixateur selon l'une des revendications 1 à 4, caractérisé en ce que
chaque articulation sphérique comprend une tête sphérique (2) qui est plaquée à l'intérieur d'une pièce de serrage de sphère (4) en forme de douille, pourvue d'un filetage extérieur (11) avec une surface d'appui pour tête sphérique (12) à incurvation sphérique, faisant saillie en forme annulaire vers l'intérieur, où
la pièce de serrage de sphère (4), avec la tête sphérique (2) placée sur elle, est vissable dans un trou taraudé (13) associé de la mâchoire de serrage (3), avec interposition d'une rondelle de pressage de sphère (5), pourvue d'une surface de serrage pour tête sphérique (14), à incurvation sphérique, rondelle prévue entre la tête sphérique (2) et la mâchoire de serrage (3).

6. Fixateur selon la revendication 5, caractérisé en ce que
le rayon (R₁; R₂) de la surface de serrage pour tête sphérique (14), à incurvation sphérique, du disque de pressage de sphère (5) et/ou de la surface d'appui pour tête sphérique (12) à incurvation sphérique, à l'intérieur de la pièce de serrage sphérique (4) en forme de douille, dans la zone du sommet, est inférieur à ce qu'il est dans la zone restante, ou va en diminuant en allant en direction du sommet.

7. Fixateur selon l'une des revendications 1 à 6, caractérisé en ce que
la tête sphérique (2) et/ou les surfaces de serrage ou d'appui (14, 12) pour la tête sphérique sont revêtues de poussière de diamant.

8. Fixateur selon l'une des revendications 1 à 7, caractérisé en ce qu'à la rondelle de pressage de sphère (5) est associée une vis de serrage (6) vissable dans la mâchoire de serrage (3), vis au moyen de laquelle l'articulation sphérique peut être engagée sur le côté de la rondelle de pressage de sphère (5) qui est opposé à la surface de serrage (14) pour tête sphérique et donc bloquée.

9. Fixateur selon la revendication 8, caractérisé en ce que la mâchoire de serrage (3) est un composant du genre d'une barre monopièce, en particulier ronde, sur une face frontale de laquelle est réalisé un trou taraudé (13) destiné à recevoir la rondelle de pressage de sphère (5), la tête sphérique (2) et la pièce de serrage de sphère (4) en forme de douille, et composant à travers lequel la vis de serrage (6) associée à la rondelle de pressage de sphère (5) s'étend axialement, les perçages transversaux (22) destinés à recevoir et fixer les clous d'ostéosynthèse (19) s'étendant chacun latéralement au perçage taraudé axial destiné à la vis de serrage (6).

10. Fixateur selon l'une des revendications 1 à 9, caractérisé en ce que chaque mâchoire de serrage (3) comporte trois perçages (22) destinés à recevoir les clous d'ostéosynthèse (19) pouvant chacun être bloqués par trois vis.

11. Fixateur selon la revendication 10, caractérisé en ce que les perçages (22) recevant les clous d'ostéosynthèse (19) ont un diamètre d'environ 6,0 mm, à 4,0 mm.

12. Fixateur selon l'une des revendications 1 à 11, caractérisé en ce que la douille (1) recevant la barre ronde (10) présente un filetage extérieur (16) sur lequel sont vissés, en association avec les organes d'entraînement reliés aux barres rondes (10), en particulier des vis (8), au moins un, en particulier deux écrous, de préférence des écrous à six pans (17, 18).

13. Fixateur selon la revendication 12, caractérisé en ce que le ou les écrous (17, 18) agisse(nt) entre les deux organes d'entraînement de barre ronde ou vis (8), et précisément en association chaque fois avec un organe d'entraînement de barre ronde ou une vis (8) relié à une barre ronde (10).
